(19) Europäisches Patentamt / European Patent Office / Office européen des brevets

(11) **EP 4 666 999 A1**

(12) **EUROPEAN PATENT APPLICATION**
published in accordance with Art. 153(4) EPC

(43) Date of publication:
**24.12.2025 Bulletin 2025/52**

(21) Application number: **24757046.8**

(22) Date of filing: **19.01.2024**

(51) International Patent Classification (IPC):
**A61K 9/00** (2006.01)   **A61K 9/06** (2006.01)
**A61K 47/10** (2017.01)   **A61K 38/39** (2006.01)
**A61P 43/00** (2006.01)

(52) Cooperative Patent Classification (CPC):
**A61K 9/00; A61K 9/06; A61K 38/39; A61K 47/10; A61P 43/00**

(86) International application number:
**PCT/KR2024/000965**

(87) International publication number:
**WO 2024/172315 (22.08.2024 Gazette 2024/34)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.02.2023 KR 20230019511**

(71) Applicant: **T&R Biofab Co., Ltd.**
**Siheung-si Gyeonggi-do 15111 (KR)**

(72) Inventors:
• **KIM, Hyun Jung**
  **Anyang-si, Gyeonggi-do 14061 (KR)**
• **LEE, Song Mi**
  **Incheon 21931 (KR)**
• **YOON, Yi Hyun**
  **Bucheon-si, Gyeonggi-do 14412 (KR)**

(74) Representative: **Caspary, Karsten**
**Kroher-Strobel**
**Rechts- und Patentanwälte PartmbB**
**Bavariaring 20**
**80336 München (DE)**

(54) **DRUG DELIVERY COMPOSITION FOR PROMOTING TISSUE REGENERATION AND KIT FOR DRUG DELIVERY COMPRISING SAME**

(57) The present invention relates to a hydrogel-based drug delivery system that can efficiently deliver the required amount of a drug to a desired location for a longer period of time, the hydrogel-based drug delivery system comprising: an amphipathic block copolymer including a mixture of a first poloxamer and a second poloxamer that have different molecular weights; and a protein extract (VdECM) containing elastin and collagen. A drug delivery composition for promoting tissue regeneration, according to the present invention, is mixed with a drug and introduced into the body, thus increasing the excretion time of the drug in the body, has phase transition properties of a liquid (sol) state at room temperature while being gelled at body temperature, thus being convenient to inject as an injectable agent, can be applied to various body parts, and comprises a protein extract (VdECM) containing an elastin component involved in a damaged tissue regeneration process, thus having the effect of rapidly recovering damaged tissue.

[FIG. 11]

**Description**

[Technical Field]

The present disclosure relates to a hydrogel-based drug delivery system capable of efficiently delivering a required amount of a drug to a target site for a long period of time, and in particular, to a drug delivery composition with excellent sustained-release properties, which increases the release time of a drug *in vivo* when mixed with a drug and administered into the body, and a kit for drug delivery comprising the same.

The drug delivery composition according to the present disclosure is easy to handle in a sol state at room temperature but undergoes a phase transition into a gel state at around body temperature. Due to its characteristic of slowly biodegrading within the body, it has sustained-release properties that allow the drug mixed therein to be continuously released for a long period of time, thereby enhancing the therapeutic effect of the drug.

Further, the present disclosure relates to a drug delivery composition for promoting tissue regeneration capable of promoting tissue regeneration by further including a protein extract (VdECM) containing elastin and collagen obtained through a multi-step decellularization process, and a kit for drug delivery comprising the same.

[Background Art]

With the advent of an aging society, there has been an increasing demand for effective treatment methods for various diseases. Moreover, due to population aging and the rising prevalence of chronic diseases, the market for drug delivery technology capable of efficiently delivering the required amount of a drug to a target site while minimizing side effects has been rapidly expanding.

In particular, various products have been commercialized as drug delivery systems or kits for drug delivery capable of delivering local anesthetics into the body for pain control. These products have the advantage of fast onset of action due to rapid absorption in the body; however, same rapid absorption also limits their ability to provide sustained pain relief for a long period of time.

To address these issues associated with local anesthetics for pain control, a variety of drug delivery systems have been commercialized.

For example, there is a method in which a catheter having multiple micro-holes is inserted into the surgical site or the surrounding nerve tissue after surgery, and is connected to an elastomeric pump to continuously administer a local anesthetic. However, since the catheter is inserted into the body for drug infusion, this method may cause pain, is prone to blockage of the catheter holes, requires removal of the catheter from the body after use, and restricts the patient's freedom of movement.

Instead of such mechanical devices, there is an implant-type method that has been developed using a combination of a bioabsorbable collagen sponge and an amide-type local anesthetic (bupivacaine HCl), and is primarily used in inguinal hernia surgery. While this method has the advantage of not requiring an additional removal procedure due to its use of a bioabsorbable material, it also has several disadvantages in that the product comes in the form of a sponge with a fixed size and shape, making it difficult to apply to areas lager than its own size, requiring it to be cut to fit the application site, and making it difficult to insert the product into the body through a narrow administration pathway.

Recently, a drug delivery system for pain control has been commercialized that encapsulates a local anesthetic (bupicacaine HCl) within a liposome, allowing for slow release of the local anesthetic for about 72 hours. The liposomes used in these drug delivery systems are small spherical phospholipid structures that encapsulate the local anesthetic, with the particles themselves slowly decomposing and being absorbed into the body. However, they are necessarily required to be stored under refrigerated conditions (2 to 8°C), making them susceptible to long-term storage. Furthermore, the low stability of liposomes makes it difficult to stably release the drug for a long period of time in the body.

[Disclosure]

[Technical Problem]

To effectively address the problems of such prior art, an object of the present disclosure is to provide a novel drug delivery composition capable of being easily administered into the body in a liquid state, forming a stable drug delivery structure through a phase transition induced by body temperature, thereby effectively releasing a drug component in the body for a long period of time, being biodegradable and absorbable in the body, and promoting tissue regeneration, and a kit for drug delivery comprising the same.

[Technical Solution]

According to an embodiment of the present disclosure, a drug delivery composition for promoting tissue regeneration comprises: an amphiphilic block copolymer including a mixture of a first poloxamer and a second poloxamer having different molecular weights; and a protein extract (VdECM) containing elastin and collagen.

The amphiphilic block copolymer is in a sol phase at room temperature and undergoes a phase transition to a gel phase at body temperature.

The drug delivery composition for promoting tissue may comprise 0.05 to 10 wt%, preferably 0.05 to 6 wt% of the protein extract (VdECM), 1 to 40 wt% of the first poloxamer, 1 to 40 wt% of the second poloxamer, and the balance of ultrapure water.

The first poloxamer preferably has a block copolymer structure of polyethylene oxide (PEO)-polypropylene oxide (PPO)-polyethylene oxide (PEO) and has a weight-average molecular weight of 5,000 to 15,000. The second poloxamer more preferably has a block copolymer structure of polyethylene oxide (PEO)-polypropylene oxide (PPO)-polyethylene oxide (PEO) and has a weight-average molecular weight of 8,000 to 20,000.

The protein extract (VdECM) included in the drug delivery composition for promoting tissue regeneration according to the present disclosure may be prepared or obtained through the following steps: a pretreatment step of preparing and pretreating tissue of non-human mammal; a first inactivation step of inactivating viruses contained in the pretreated tissue using an alcohol; a decellularization step of removing cells from virus-inactivated tissue; and a second inactivation step of inactivating viruses contained in the decellularized tissue using an acid.

It is preferable that a DNA content of the tissue that had undergone to the second inactivation step is 50 ng/mg or less, and that a reduction rate (L) of the elastin content in the tissue that had undergone the pretreatment to second inactivation steps is 20% or less.

The decellularization step may include a primary decellularization step of removing cells from the virus-inactivated tissue using an alkaline aqueous solution; and a secondary decellularization step of removing cells by enzymatic treatment of the primarily decellularized tissue. The primary decellularization step may include a first decellularization step performed using a mixture of n-PrOH and NaOH; and a second decellularization step performed using an aqueous sodium hydroxide solution having a concentration of greater than 0.05 M and less than 0.2 M.

The tissue of a non-human mammal used in the present disclosure is preferably one or more selected from the group consisting of blood vessels, ligaments, and tendons derived from a mammal.

The secondary decellularization step is preferably performed using DNase.

In another embodiment of the present disclosure, there is provided a kit for drug delivery in the form of a syringe or injector filled with the drug delivery composition for promoting tissue regeneration as described above.

In still another embodiment of the present disclosure, there is provided a drug delivery system for promoting tissue regeneration, comprising a mixture of the drug delivery composition for promoting tissue regeneration according to an embodiment and a drug at a weight ratio of 1:1.

[Advantageous Effects]

The drug delivery composition for promoting tissue regeneration according to the present disclosure comprises poloxamer, which is a temperature-sensitive material, and has phase transition properties of a liquid (sol) state at room temperature while being gelled at body temperature, thereby allowing for convenient injection as an injectable formulation and enabling application to various sites of the body.

In addition, the drug delivery composition for promoting tissue regeneration comprise a protein extract (VdECM) containing elastin, which is involved in the regeneration process of damaged tissue, thereby contributing to the rapid recovery of the damaged tissue.

Moreover, elastin, composed of 70% or more hydrophobic amino acids, may bind with poloxamer, which is an amphiphilic temperature-sensitive polymer, which has both hydrophilic and hydrophobic properties, thereby enhancing *in vivo* stability and tissue adhesion. As a result, after being injected into the body via syringe, the composition remains stably at the injection site without migrating, thereby enabling effective localized drug delivery.

In particular, since poloxamer and the protein extract (VdECM) are bioabsorbable within 14 days, no separate removal process is required. By adopting amphiphilic poloxamer, which has both hydrophobic and hydrophilic properties, both hydrophilic and hydrophobic drugs may be used. As a result, they can be used in combination with a variety of drugs, such as growth factors, steroids, antibiotics, analgesics, and topical anticancer agents, and allow for uniform mixing with a variety of drugs.

[Description of Drawings]

FIGS. 1(A) and 1(B) are graphs showing the experimental results of Experimental Example 1.

FIG. 2 is a graph showing the experimental results of Experimental Example 2.

FIGS. 3(A) and 3(B) are graphs showing the experimental results of Experimental Example 3.

FIGS. 4(A) and 4(B) are graphs showing the experimental results of Experimental Example 4.

FIG. 5 is a table showing the experimental results of Experimental Example 5.

FIG. 6 shows the results of testing cytotoxicity according to changes in the concentration of the protein extract in the drug delivery composition of the present disclosure.

FIGS. 7 and 8 show the results of measuring changes in viscosity according to changes in the concentration of the protein extract in the drug delivery composition of the present disclosure.

FIG. 9 shows the results of measuring changes in adhesive strength according to changes in the concentration of the protein extract in the drug delivery composition of the present disclosure.

FIG. 10 shows the results of measuring gel stability over time according to changes in the concentration of the protein extract in the drug delivery composition of the present disclosure.

FIG. 11 shows the results of measuring the amount of drug released over time in order to confirm the drug delivery performance of the present disclosure.

[Best Mode]

Before describing the present disclosure in more detail below with reference to preferred embodiments of the present disclosure, it should be noted that the terms and words used in this specification and claims are not to be construed in their ordinary or dictionary sense, but rather in a sense and concept consistent with the technical ideas of the present disclosure.

Throughout the specification, "comprising" any component will be understood to imply the inclusion of other components rather than the exclusion of other components, unless otherwise specifically stated.

In addition, throughout the specification, "%" used to indicate a concentration of a particular substance refers to (weight/weight) % for solid/solid, (weight/volume) % for solid/liquid, and (volume/volume) % for liquid/liquid, unless otherwise stated.

The term "mammal" as used herein refers to a non-human mammal, and even where simply referred to as "mammal" without specifying the exclusion of humans, it should be understood to mean a non-human mammal. The expression "cells are removed" should be understood broadly to include cases in which DNA is destroyed or removed.

In addition, the respective steps may be performed in an order different from that explicitly described, unless the context clearly dictates a specific sequence. That is, the respective steps may be performed in the same order as stated, substantially simultaneously, or in reverse order.

Unless otherwise defined, all technical and scientific terms as used herein have the same meanings as commonly understood by those skilled in the art to which the present disclosure pertains. In the case of conflict, the description in the present specification of the present disclosure, including definitions, will prevail.

A drug delivery composition for promoting tissue regeneration according to an embodiment of the present disclosure comprises an amphiphilic block copolymer; and a protein extract (VdECM) containing elastin and collagen. The amphiphilic block copolymer may include a mixture of a first poloxamer and a second poloxamer having different molecular weights. The protein extract (VdECM) is obtained through a multi-step decellularization process from tissue of a non-human mammal, such as blood vessels, ligaments, and tendons derived from a mammal. The protein extract (VdECM) contains about 60% elastin, 40% collagen, and trace amounts of growth factors, and bioactive substances.

The drug delivery composition for promoting tissue regeneration may comprise 0.05 to 10 wt%, preferably 0.05 to 6 wt% of a protein extract (VdECM), 1 to 40 wt% of each of the first and second poloxamers, and the balance preferably comprising ultrapure water as a solvent.

To dissolve the protein extract (VdECM) in powder form, an alkaline aqueous solution containing a basic substance at a concentration of 0.01 to 10 N in ultrapure water was used. After the protein extract was dissolved, it is more preferable to use a neutral solvent based on ultrapure water, which has been neutralized using an organic acid to a neutral range, preferably to a pH value in the range of 7.0 to 7.5.

Examples of the basic substances used here include sodium hydroxide, potassium hydroxide, ammonia, sodium carbonate, sodium bicarbonate, potassium carbonate, and potassium bicarbonate. Examples of organic acids used to neutralize the alkaline aqueous solution containing the dissolved protein extract include 0.01 to 10 N of acetic acid, lactic acid, hydrochloric acid, citric acid, succinic acid, glycolic acid, perchloric acid, carboxylic acid, and sulfonic acid.

After dissolving the protein extract powder in the alkaline aqueous solution, the solution is neutralized to a neutral range using an organic acid. Then, a primary filtration process is performed, followed by the dissolution of the first and second poloxamers. The drug delivery composition for promoting tissue regeneration according to the present disclosure may be prepared by dissolving the first and second poloxamers, performing a low-temperature, low-speed stirring and/or reduced pressure treatment, and then performing a defoaming process to remove air bubbles within the composition, followed by packaging and sterilization.

The drug delivery composition may be packaged in a first syringe, which is a syringe-shaped container, and a drug to be used in combination with the drug delivery composition may also be filled in a separate second syringe. Then, the discharge ports of the first and second syringes may be connected to each other via a connection member to allow the drug delivery composition and the drug to be pre-mixed before being injected or applied to the affected area or application site.

Elastin plays a role in maintaining shape or form and providing elasticity in the extracellular matrix (ECM), as well as in regulating intercellular signaling by binding to elastin binding sites (the elastin receptor complex (ERC), GAGs, and the integrin $\alpha$V$\beta$3) present on the cell membrane.

The main roles of elastin include cell signaling, migration, attachment, proliferation, survival, development, differentiation, phenotype, ECM production, and regulation of physiology. Elastin is also involved in promoting re-epithelialization, damaged tissue regeneration, angiogenesis, and MMP-1 expression. Thus, elastin plays a vital role in maintain tissue structure and regulating physiological activity in normal tissues. Moreover, when elastin components are externally administered to damaged tissue, they may promote the proper formation of elastic fiber tissue, thereby restoring the tissue to a state similar to its original condition.

As such, elastin may play various roles in the recovery or healing process of damaged tissue. For example, during the process where an injured blood vessel constricts to achieve hemostasis, elastin promotes the migration of inflammatory cells to the injury site and the release of related factor. During the process of removing necrotic tissue from the injury site, elastin promotes the migration, adhesion, proliferation, and differentiation of epithelial cells at the wound sit. In addition, elastin promotes the proliferation of cells and extracellular matrix at the injury site, and participates in the synthesis of collagen and elastin, which form the structural framework essential for wound healing. This promotes angiogenesis and elastin expression, thereby restoring tissue with elasticity similar to that of the native tissue.

Meanwhile, the amphiphilic block copolymer included in the drug delivery composition preferably includes a mixture of a first poloxamer and a second poloxamer having different molecular weights. The first poloxamer preferably has a weight-average molecular weight of about 5,000 to 15,000, and the second poloxamer preferably has a weight-average molecular weight of about 8,000 to 20,000. In addition, the first and second poloxamers may each be included in an amount of 1 to 40 wt% in the drug delivery composition, and by using a mixture of two types of poloxamers having different molecular weights as the amphiphilic block copolymer, *in vivo* stability and ease of use are improved compared to those achieved by using a single poloxamer.

That is, to prepare a formulation capable of being maintained for about 7 days or more using a single poloxamer, a high concentration of the poloxamer is required. However, the formulation designed to achieve high phase stability suffers from poor user convenience due to high viscosity at room temperature, and thus is unsuitable for use as drug delivery compositions.

However, in the present disclosure, by mixing two different types of poloxamer and a protein extract (VdECM), an injectable formulation is obtained that remains in a liquid state at room temperature, making it convenient for injection using a syringe. In addition, after injection into the body, the phase may transform into a solid gel as the temperatures rises due to body temperature, thereby maintaining high phase stability for a long period of time, about 7 days or more.

The amphiphilic block copolymer possesses both hydrophilic and hydrophobic polymer properties. Therefore, after the hydrogel is formed, when a poorly soluble hydrophobic drug is applied, the interaction between the hydrophobic groups allows the drug to be homogenized within the hydrogel. In addition, hydrophilic drugs may also be easily homogenized within the hydrogel.

Poloxamers and poloxamines, representative amphiphilic block copolymers, are composed of hydrophilic polyethylene oxide (PEO) blocks and hydrophobic polypropylene oxide (PPO) blocks. They are effective in encapsulating both hydrophobic and hydrophilic drugs, and exhibit reversible sol-gel phase transition properties at specific temperatures, providing convenience in use. Furthermore, they exhibit excellent biocompatibility, are non-ionic and non-reactive, and therefore suitable for use as drug delivery materials.

On the other hand, in the case of a single-component amphiphilic block copolymer, gelation occurs at relatively high concentrations, and it is rapidly absorbed and excreted from the body within a few hours after injection into the body. Therefore, there are limitations in maintaining a sustained release of the drug at a constant concentration over a certain period while retaining it within the body for a long period of time.

It is preferable that the first poloxamer has a structure of polyethylene oxide (PEO)-polypropylene oxide (PPO)-polyethylene oxide (PEO) and has a weight-average molecular weight of 5,000 to 15,000. It is also preferable that the first poloxamer is an amphiphilic copolymer comprising polypropylene oxide (PPO) having a weight-average molecular weight of 4,000 and about 50 to 75 wt% of polyethylene oxide (PEO) and having an HLB value of 24 or less.

It is preferable that the second poloxamer also has a structure of polyethylene oxide (PEO)-polypropylene oxide (PPO)-polyethylene oxide (PEO) and has a weight-average molecular weight of 8,000 to 20,000. The second poloxamer may be an amphiphilic copolymer comprising polypropylene oxide (PPO) having a weigh-average molecular weight of 3,300 and about 55 to 85 wt% of polyethylene oxide (PEO) and having an HLB value of 24 or more.

Next, the protein extract (VdECM) obtained through a multi-step decellularization process will be described in more detail. The protein extract (VdECM) included in the drug delivery composition for promoting tissue regeneration according to the present disclosure may be obtained through the following steps: a preparation step of preparing tissue of a non-human mammal; a pretreatment step of pretreating the tissue; a first inactivation step of inactivating viruses contained in the pretreated tissue using an alcohol; a primary decellularization step of removing cells from virus-inactivated tissue using an alkaline aqueous solution; a secondary decellularization step of removing cells by enzymatic treatment of the primarily decellularized tissue; and a second inactivation step of inactivating viruses contained in the decellularized tissue using an acid.

Here, the DNA content of the tissue that had undergone the second inactivation step may be 50 ng/mg or less, preferably 20 ng/mg or less, 10 ng/mg or less, and more preferably 5 ng/mg or less. In addition, it is more preferable that a reduction rate (L) of elastin content in the tissue that had undergone the pretreatment step to the second inactivation step is 20% or less.

Here, the reduction rate (L) is defined by the following Equation (1), where $L_0$ represents the elastin content in the tissue during the preparation step, and $L_f$ represents the elastin content in the tissue that had undergone the second inactivation step.

$$L = \frac{(L_o - L_f)}{L_o} \times 100 \qquad \text{(Equation 1)}$$

The preparation step is a step of preparing tissue of a non-human mammal. Here, mammals refer to non-human mammals, for example, pigs, horses, cattle, sheep, etc., and the tissue may be one or more selected from the group consisting of blood vessels, ligaments, and tendons derived from such mammals.

To prepare the multi-step decellularized protein extract (VdECM), tissue is first harvested from a mammal. The harvested mammalian tissue is then prepared during the preparation step by removing unnecessary tissues and blood adhered to the mammalian tissue, followed by washing, drying, and cutting. The prepared mammalian tissue may be stored in a frozen state and thawed for use when operation is required. When frozen tissue raw materials are used, the preparation step may be a step of taking out the frozen tissue raw materials and preparing them.

Next, the mammalian tissue prepared from the preparation step is pretreated through the pretreatment step. This step is a step of washing the mammalian tissue, and when the mammalian tissue is in a frozen state, thawing, washing, and cutting may be performed during this step. In this step, distilled water, purified water, saline solution, etc. may be used as washing water, preferably distilled water. From this step to the second inactivation step, physical agitation may be applied.

The mammalian tissue prepared through the pretreatment step undergoes a first inactivation step, a primary decellularization step, a secondary decellularization step, and a second inactivation step, during which cells, neutral fat, viruses, and other foreign substances that may induce immune responses and foreign body reactions may be removed. By performing decellularization through this multi-step process, decellularization efficiency and foreign substance removal efficiency at each step are significantly improved, thereby increasing the amount of tissue processed at one time and shortening treatment time. As a result, the productivity and yield of the decellularized protein extract (VdECM) may be improved.

First, the first inactivation step is a step of inactivating viruses contained in the pretreated tissue using an alcohol.

This step is performed prior to decellularization of the tissue to primarily inactivate viruses in the tissue, thereby enabling more effective removal of cells, neutral fact, and foreign substances during the primary and secondary decellularization steps.

The alcohol used in this step may be n-propanol. To achieve a virus inactivation effect, an aqueous n-propanol solution at a concentration of 50 to 90% is preferably used, and more preferably, an aqueous n-propanol solution at a concentration of 65 to 80% may be used.

In the present disclosure, n-propanol is used as the alcohol for virus inactivation in the first inactivation step. When n-propanol is used for virus inactivation, the tissue becomes softer, which allows a treatment solution to penetrate more effectively into the tissue, resulting in uniform virus inactivation and decellularization overall. As a result, the efficiency of inactivation and decellularization treatment may be improved, more uniform tissues may be obtained, and the quality and reliability of the final obtained decellularized protein extract (VdECM) may be improved.

When ethanol, which is generally used for virus inactivation, is used, it has a characteristic of fixing and hardening the mammalian tissue to be decellularized. When ethanol is used instead of n-propanol in this step of the present disclosure, the tissue hardens, thereby reducing the efficiency of the process that loosen the tissue in subsequent steps to improve penetration of the treatment substances. Therefore, the use of ethanol is not preferable.

In addition, since iso-propanol has a lower virus inactivation effect, when iso-propanol is used in the first inactivation step, sufficient virus inactivation is not achieved, resulting in reduced decellularization efficiency in the subsequent primary and secondary decellularization steps. Thus, it is preferable to use n-propanol in the first inactivation step.

In this step, 10 to 25 parts by weight, and preferably 20 to 25 parts by weight, of the pretreated tissue may be treated per 100 parts by weight of the propanol aqueous solution.

The primary decellularization step is a step of removing cells by treating the virus-inactivated tissue that had undergone the first inactivation step, with an alkaline aqueous solution.

This step includes a first decellularization step of treating the tissue using a mixture of an alkaline aqueous solution and an alcohol; and a second decellularization step of treating the tissue using an alkaline aqueous solution. Here, the alkaline aqueous solution is preferably an aqueous sodium hydroxide solution, and the alcohol is preferably n-propanol. In this case, the first decellularization step is a step of treating the tissue with a mixture of n-propanol and sodium hydroxide, and the second decellularization step is a step of treating the tissue with an aqueous sodium hydroxide solution.

As such, when the decellularization process is performed in two steps, the first decellularization step not only removes decellularized tissue but also removes foreign substances such as neutral fat, resulting in a more effective decellularization process in the second decellularization step.

In each of the first and second decellularization steps, 10 to 25 parts by weight, and preferably 20 to 25 parts by weight, of the tissue may be treated per 100 parts by weight of the treatment solution. This represents approximately twice the conventional treatment capacity. Since the multi-step method of the present disclosure enables effective decellularization, it allows sufficient treatment of twice the volume of tissue compared to conventional methods.

Specifically, the first decellularization step is a step of treating the virus-inactivated tissue with a mixture of n-propanol and sodium hydroxide. During this step, the neutral fat within the tissue undergoes a saponification reaction with sodium hydroxide, resulting in the separation and removal of the neutral fat from the tissue. Here, n-propanol promotes the saponification reaction and acts to aggregate the product of saponification reaction, allowing the neutral fat to be separated and removed from the tissue more quickly and efficiently.

Here, the treatment time for treating the tissue with a mixture of n-propanol and sodium hydroxide may be 10 to 48 hours, preferably 15 to 40 hours, and more preferably 18 to 28 hours. The treatment temperature may be 0 to 30°C, preferably 10 to 28°C, and more preferably 18 to 25°C. When the treatment is conduced within this temperature range and for the above treatment time, sufficient removal efficiency of neutral fat may be achieved without significant damage to the tissue structure or loss of elastin.

The mixture of n-propanol and sodium hydroxide may be an aqueous solution wherein the concentration of n-propanol may be 50 to 95%, preferably 60 to 90%, and more preferably 65 to 85%, and the concentration of sodium hydroxide may be greater than 0.05 M and less than 0.2 M. These concentration ranges may minimize tissue damage during the first decellularization step while simultaneously effectively removing foreign substances such as neutral fat. In particular, when the concentration of sodium hydroxide is below the above range, the treatment efficiency of the primary decellularization step decreases. When the concentration of sodium hydroxide exceeds the above range, the tissue structure itself may be collapsed and dissolved. Therefore, it is preferable to use n-propanol and an aqueous sodium hydroxide solution within the concentration range as described above.

The second decellularization step is a step of treating the tissue that had undergone the first decellularization step with an alkaline aqueous solution to soften the tissue, thereby loosening its structure while simultaneously inducing the decellularization reaction. Neutral fat and various foreign substances contained in the tissue serve as a kind of physical and chemical barriers to the decellularization reaction. Because the tissue has already undergone the first decellularization step and had been cleared of neutral fat and other foreign substances, the decellularization reaction may be performed more effectively in the second decellularization step.

In this step, the treatment time for treating the tissue with an alkaline aqueous solution may be 10 to 48 hours, preferably 15 to 40 hours, and more preferably 18 to 28 hours. The treatment temperature may be 0 to 30°C, preferably 10 to 28°C, and more preferably 18 to 25°C. When the treatment is conduced within this temperature range and for the above treatment time, the tissue structure may be sufficiently loosened without collapse of the tissue structure or loss of elastin, and an appropriate decellularization reaction may occur.

Here, the alkaline aqueous solution used as the treatment solution may be an aqueous sodium hydroxide solution, and the concentration of sodium hydroxide contained in the aqueous sodium hydroxide solution may be greater than 0.05 M and less than 0.2 M. When the concentration of sodium hydroxide is 0.05 M or less, the tissue structure may be insufficiently loosened, resulting in reduced decellularization efficiency in the secondary decellularization step described below. When the concentration of sodium hydroxide is 0.2 M or more, some tissues may disintegrate or dissolve beyond mere loosening during this step, resulting in a significant decrease in final yield and loss of elastin. Therefore, it is preferable to use an aqueous sodium hydroxide solution within the concentration range as described above.

After the primary decellularization step, a secondary decellularization step using enzymes is performed, and a neutralization step and a washing step may be performed between these two steps.

The neutralization step is a step of treating the tissue with an acidic aqueous solution to neutralize the sodium hydroxide used in the primary decellularization step. The acidic aqueous solution used in this step may include, but is not limited to, one or more of hydrochloric acid, sulfuric acid, acetic acid, and peracetic acid. The concentration of these acidic aqueous solutions may also be appropriately adjusted according to the working environment or conditions.

The washing step is a step of preventing a decrease in enzymatic reactivity due to residues during the enzymatic treatment in the subsequent secondary decellularization step. A buffer solution may be used as the washing solution in the washing step. Examples thereof include, but are not limited to, phosphate buffered saline (PBS).

Meanwhile, the secondary decellularization step is a step of removing cells by enzymatic treatment of the primary decellularized tissue, and specifically, is as step of removing DNA within the primary decellularized tissue by treating the primary decellularized tissue with DNase.

In this step, 10 to 25 parts by weight, preferably 20 to 25 parts by weight, of the tissue may be treated per 100 parts by weight of the treatment solution containing DNase. As previously described, this represents approximately twice the conventional treatment capacity, and is achievable due to the increased treatment efficiency achieved by the multi-step decellularization process of the present disclosure.

In this step, to obtain enzyme activity and sufficient DNA degradation efficiency, the treatment time for treating the tissue with DNase may be 10 to 35 hours and preferably 18 to 30 hours. The treatment temperature may be 30 to 45°C and preferably 35 to 42°C.

In the present disclosure, the concentration of DNase included in the treatment solution is 0.0001 to 0.005 wt%, which is several to hundreds of times lower than the concentration of DNase typically used in similar prior art. The primary decellularized tissue of the present disclosure undergoes a primary decellularization step, during which foreign substances, including neutral fat, within the tissue are removed and the tissue structure becomes loose. When DNase is administered in this state, it may easily penetrate the tissue structure. Therefore, even when a low concentration of DNase is used, at least the same or better DNA removal efficiency may be secured, so in the present disclosure, a low concentration of DNase as described above may be used.

However, when the concentration of DNase is below the above range, DNA degradation efficiency decreases, so it is preferable to use a concentration of 0.0001% or more. When the concentration of DNase exceeds the above range, the improvement in DNA degradation efficiency is extremely minimal compared to the amount of DNase added, making it uneconomical. Thus, it is preferable to use DNase within the concentration range as described above.

The tissue decellularized through the secondary decellularization step undergoes a second inactivation step, during which additional viral removal is performed, and a decolorization step and/or a defatting step may additionally be performed between these two steps. The bleaching step is a step of removing the color of the tissue, and may be performed, for example, by treating the tissue with hydrogen peroxide. The defatting step is a step of additionally removing lipids contained in the tissue, and may be performed, for example, using a ketone solution, preferably an acetone solution.

Then, a second inactivation step is performed to further inactivate viruses contained in the tissue that had undergone the secondary decellularization step. This step is a step of inactivating viruses in the tissue using an acid, and specifically, is a step of treating the tissue with a mixture of an organic acid an and an alcohol.

The organic acid used in this step may be peracetic acid. Peracetic acid is an oxidizing agent that may inactivate viruses by inducing non-specific free radical damage. Peracetic acid used in this step may be at a concentration of 0.05 to 1%.

Also, the alcohol used in this step may be a lower alcohol with 1 to 4 carbon atoms, such as methanol, ethanol, or propanol, preferably ethanol. Since ethanol functions to inactivate viruses and to fix the tissue structure, thereby hardening the tissue, it is preferable to use ethanol.

Through these steps, viruses may be ultimately inactivated, neutral fat and DNA may be removed, and a protein extract (VdECM) containing various extracellular matrix components, including elastin, may be obtained.

The protein extract (VdECM) thus obtained has a very low DAN content of 50 ng/mg or less, preferably 20 ng/mg or less, 10 ng/mg or less, and more preferably 5 ng/mg or less, which may minimize the induction of immune responses or various foreign body reactions.

In addition, the protein extract (VdECM) has the advantage of exhibiting effects such as wound healing and scar suppression attributable to elastin, as the reduced elastin content is very low through the pretreatment step to the second inactivation step. Specifically, the reduction rate (L) of elastin content in the tissue that had undergone the pretreatment step to the second inactivation step is 20% or less. This numerical value is significantly lower compared to those observed when a conventional decellularization method is applied, in which the reduction rate (L) of elastin content is very high, at 50% or more.

Meanwhile, after the second inactivation step, additional steps such as washing, packaging, sterilization, and processing may be performed. Washing may be performed using a buffer solution, distilled water, etc., and packaging may be performed to prevent contamination during storage or transportation of the protein extract (VdECM) and to facilitate handling. The protein extract (VdECM) may also be packaged while immersed in a preservation solution.

Sterilization is performed to remove any additional contamination that occurs after the second inactivation step, and may be performed, for example, using, but is not limited to, electron beam irradiation or radiation irradiation.

Processing refers to prepare the protein extract (VdECM) into a usable form. For example, methods such as drying, pulverizing, and solubilizing may be applied, and the resulting product may be utilized in forms such as bio-inks or injectable formulations. The anti-adhesion coating composition for promoting tissue regeneration according to the present disclosure may be prepared in a liquid injectable formulation for ease of handling and use.

Meanwhile, another embodiment of the present disclosure relates to a method for preparing a mammalian-derived protein extract (VdECM). Some overlapping descriptions from an embodiment of the present disclosure will be omitted.

Specifically, the method for preparing a decellularized protein extract (VdECM) includes: a preparation step of preparing tissue of a non-human mammal; a pretreatment step of pretreating the tissue; a first inactivation step of inactivating viruses contained in the pretreated tissue using an alcohol; a primary decellularization step of removing cells from virus-inactivated tissue using an alkaline aqueous solution; a secondary decellularization step of removing cells by enzymatic treatment of the primarily decellularized tissue; and a second inactivation step of inactivating viruses contained in the decellularized tissue using an acid.

Here, the DNA content of the tissue that had undergone the second inactivation step may be 50 ng/mg or less, and the reduction rate (L) of elastin content in the tissue that had undergone the preprocessing step to the second inactivation step may be 20% or less.

Here, the reduction rate (L) of elastin content in the tissue is defined by the following Equation (1), where $L_0$ represent the elastin content in the tissue during the preparation step, and $L_f$ represents the elastin content in the tissue that had undergone the second inactivation step.

$$L = \frac{(L_o - L_f)}{L_o} \times 100 \qquad \text{(Equation 1)}$$

First, the preparation step is a step of preparing tissue of a non-human mammal. Here, mammals refer to non-human mammals, for example, pigs, horses, cattle, sheep, etc., and the tissue may be one or more selected from the group consisting of blood vessels, ligaments, and tendons derived from such mammals.

The preprocessing step is a step of washing the mammalian tissue prepared in the preparation step, and, If necessary, the tissue may be cut into an appropriate size during this step. When the tissue has been prepared in a frozen state, thawing may be performed in this step.

The first inactivation step is a step of inactivating viruses contained in the preprocessed tissue using an alcohol. While ethanol is generally used for virus inactivation, the present disclosure uses n-propanol. This is because ethanol hardens and fixes the tissue, thereby reducing the softening efficiency in subsequent steps, whereas n-propanol softens the tissue, thereby improving the softening efficiency in subsequent steps.

The primary decellularization step is a step of removing cells by treating the virus-inactivated tissue that had undergone the first inactivation step, with an alkaline aqueous solution. This step includes a first decellularization step of treating the tissue using a mixture of an alkaline aqueous solution and an alcohol; and a second decellularization step of treating the tissue using an alkaline aqueous solution.

Here, the alkaline aqueous solution is preferably an aqueous sodium hydroxide solution, and the alcohol is preferably n-propanol.

Specifically, the first decellularization step is a step of treating virus-inactivated tissue with a mixture of n-propanol and sodium hydroxide. Through this step, neutral fat and foreign substances in the tissue are removed, and the tissue is partially softened. The second decellularization step is a step of treating the tissue with an aqueous sodium hydroxide solution, during which tissue softening and decellularization occur simultaneously. Since foreign substances including neutral fat have been removed in the preceding steps, these effects may be further enhanced.

In the first and second decellularization steps, sodium hydroxide may be included at a concentration of greater than 0.05 M and less than 0.2 M. This is the preferred concentration range for sufficient saponification and softening by sodium hydroxide while preventing tissue disintegration or dissolution.

Next, after the primary decellularization step, a secondary decellularization step using enzymes is performed. A neutralization step and a washing step may be performed between these two steps. The neutralization step is a step of treating the tissue with an acidic aqueous solution to neutralize the sodium hydroxide used in the primary decellularization step. The washing step is a step of preventing a decrease in enzymatic reactivity due to residues during the enzymatic treatment in the subsequent secondary decellularization step.

The secondary decellularization step is a step of removing DNA within the tissue by treating the primary decellularized tissue with DNase. The primary decellularized tissue of the present disclosure undergoes a primary decellularization step, during which foreign substances, including neutral fat, within the tissue are removed and the tissue structure becomes loose. When DNase is administered in this state, it may easily penetrate the tissue structure. Therefore, even when a low concentration of DNase is used, at least the same or better DNA removal efficiency may be secured, so in the present disclosure, DNase is used at a low concentration of 0.0001 to 0.005 wt%.

When the concentration of DNase is below the above range, DNA degradation efficiency decreases. When the concentration of DNase exceeds the above range, the improvement in DNA degradation efficiency is extremely minimal compared to the amount of DNase added, making it uneconomical. Thus, it is preferable to use DNase within the concentration range as described above.

The tissue decellularized through the secondary decellularization step undergoes a second inactivation step, during which additional viral removal is performed, and a decolorization step and/or a defatting step may additionally be performed between these two steps.

The bleaching step is a step of removing the color of the tissue, and may be performed, for example, by treating the tissue with hydrogen peroxide. The defatting step is a step of additionally removing lipids contained in the tissue, and may be performed, for example, using a ketone solution, preferably an acetone solution.

Then, a second inactivation step is performed to further inactivate viruses contained in the tissue that had undergone the secondary decellularization step. This step is a step of treating the tissue with a mixture of an organic acid and an alcohol. Peracetic acid may be used as the organic acid, and ethanol may be used as the alcohol. By treating the tissue with this mixed solution, viruses in the tissue may be effectively removed.

Through these steps, viruses may be ultimately inactivated, neutral fat and DNA may be removed, and a protein extract (VdECM) containing various extracellular matrix components, including elastin, may be prepared.

The protein extract (VdECM) thus obtained has a very low DAN content of 50 ng/mg or less, preferably 20 ng/mg or less, 10 ng/mg or less, and more preferably 5 ng/mg or less, which may minimize the induction of immune responses or various foreign body reactions.

In addition, the protein extract (VdECM) has the advantage of exhibiting effects such as wound healing and scar suppression attributable to elastin, as the reduced elastin content is very low through the pretreatment step to the second inactivation step. Specifically, the reduction rate (L) of elastin content in the tissue that had undergone the pretreatment step to the second inactivation step is 20% or less. This numerical value is significantly lower compared to those observed when a conventional decellularization method is applied, in which the reduction rate (L) of elastin content is very high, at 50% or more.

Meanwhile, after the second inactivation step, additional steps such as washing, packaging, sterilization, and processing may be performed. Washing may be performed using a buffer solution, distilled water, etc., and packaging may be performed to prevent contamination during storage or transportation of the protein extract (VdECM) and to facilitate handling. Sterilization is performed to remove any additional contamination that occurs after the second inactivation step.

Hereinafter, specific examples of the present disclosure will be described. However, the scope of the present disclosure is not limited to the following preferred examples, and those skilled in the art can implement various modified forms within the scope of the present disclosure as described in the specification.

**[Preparation Example 1]**

1. Preparation and Preprocessing Step

First, porcine heart-connected aorta, which had been washed, dried, cut, and frozen, was prepared. After thawing, the tissue was washed with distilled water and cut into specimens of 50 x 50 mm in size to prepare raw tissue specimens.

2. First Inactivation Step

Next, 250 g of raw tissue specimen was added to 1 L of 70% n-propanol and stirred at 150 RPM for 24 hours at a temperature of 20°C to perform the first deactivation step.

3.1 Primary Decellularization Step (First Decellularization Step)

Subsequently, a mixed solution containing 70% n-propanol and 0.1 M sodium hydroxide in distilled water was prepared. The raw tissue specimens were mixed at 23 parts by weight per 100 parts by weight of the mixed solution. Then, the mixture was stirred at 150 RPM for 24 hours at a temperature of 20°C to perform the first decellularization step.

3-2. Primary Decellularization Step (Second Decellularization Step)

Next, the second decellularization step was performed by mixing 24 parts by weight of the tissue specimen that had undergone the first decellularization step, with 100 parts by weight of a pre-prepared 0.1 M aqueous sodium hydroxide solution, and stirring the resulting mixture for 24 hours under the same conditions as in the previous step.

Subsequently, the tissue specimens that had undergone the second decellularization step were immersed in an aqueous acetic acid solution and stirred for neutralization, followed by washing with a PBS solution.

4. Secondary Decellularization Step

Then, the tissue specimens were added to a treatment solution containing 0.00011 wt% DNase and stirred at 150 RPM for 23 hours at a temperature of 37°C to perform the secondary decellularization step. The tissue specimens that had undergone the secondary decellularization step were subjected to a decolorization treatment by immersing it in 3% hydrogen peroxide at 20°C for 1 hour, followed by a defatting treatment by immersing it in a 20% aqueous acetone solution at 20°C for 1 hour.

5. Second Inactivation Step

Subsequently, the tissue specimens were immersed in a mixed solution containing 70% n-propanol and 0.2% peracetic acid in distilled water, and treated at 20°C for 4.5 hours to perform a second inactivation step.
Finally, the tissue specimens, which had undergone all of the above treatments, were washed with PBS solution and distilled water to prepare a multi-step decellularized protein extract (VdECM) according to an embodiment of the present disclosure.

[Experimental Example 1]

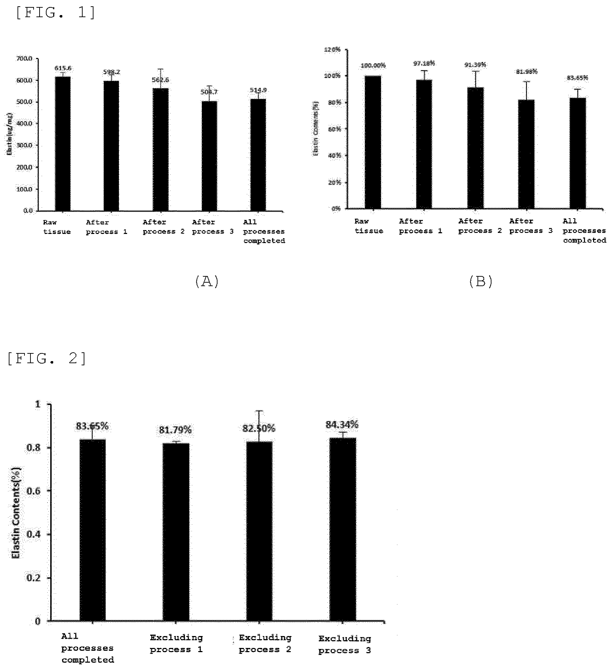

A multi-step decellularized protein extract (VdECM) was prepared using the same method as in Preparation Example 1, except that the first, second, and secondary decellularization steps were sequentially performed, and tissue specimens were harvested immediately after each step to measure the elastin content per 1 mg of tissue specimen. The results are shown in FIG. 1(A).
In addition, the elastin content of the original tissue was measured during the preparation step. The elastin content at each step was expressed as a percentage relative to the elastin content of the original tissue. The results are shown in FIG. 1(B).
In FIGS. 1(A) and 1(B), Process 1 refers to the first decellularization step, Process 2 refers to the second decellularization step, and Process 3 refers to the secondary decellularization step.
The elastin content was measured by performing an assay using a Fastin Elastin Assay kit F2000 (manufacturer: Biocolor) in accordance with the assay kit protocol, analyzing elastin by measuring absorbance at 513 nm using a Multimode Plate Reader Victor Nivo™ (manufacturer: PerkinElmer), and determining the elastin content by comparison with a standard substance.
Referring to the results of FIGS. 1(A) and 1(B), it could be confirmed that a slight loss of elastin content occurred as each process progressed, but about 80% or more of elastin remained at the end of the final process.

[Experimental Example 2]

A multi-step decellularized protein extract (VdECM) was prepared using the same method as in Preparation Example 1, except that one of the first, second, and secondary decellularization steps was omitted in each case. Tissue specimens that had undergone the final process were harvested and the elastin content per 1 mg of tissue specimen was measured. The elastin content of the original tissue was measured using the same method in the preparation step, and the L value was calculated. The results are shown in Table 1.
Additionally, the elastin content included in the protein extracts (VdECM) obtained by omitting each step was calculated as a percentage relative to the elastin content of the original tissue in the preparation step. The results are shown in FIG. 2.
The elastin content was measured using the same method as in Experimental Example 1. In Table 1 and FIG. 2, Process 1 refers to the first decellularization step, Process 2 refers to the second decellularization step, and Process 3 refers to the secondary decellularization step.

[Table 1]

|  | All processes completed | Except for Process 1 | Except for Process 2 | Except for Process 3 |
|---|---|---|---|---|
| LO (μg/mg) | 615.6 | | | |
| Lf (μg/mg) | 514.9 | 503.5 | 507.8 | 519.1 |
| L (%) | 16.4 | 18.2 | 17.5 | 15.7 |

First, referring to Table 1, it can be confirmed that the L value was 20% or less both when all processes are performed and when one process is omitted. Additionally, referring to FIG. 2, it could be confirmed that the elastin content did not significantly differ even if a specific process is omitted. Therefore, based on the results of this experiment, it can be confirmed that omission of any one of the first decellularization step, the second decellularization step, and the secondary decellularization step in the process according to an embodiment of the present disclosure did not significantly affect the elastin content.

**[Experimental Example 3]**

The experiment was conducted using the same method as in Experimental Example 1, except that DNA content was measured instead of elastin content after each step. The DNA content was measured by performing an assay using a Quant-iT™ PicoGreen™ dsDNA Assay kit and dsDNA Reagents (manufacturer: Invitrogen) in accordance with the assay kit protocol and measuring absorbance at 480 to 520 nm using a Multimode Plate Reader Victor Nivo™ (manufacturer: PerkinElmer).

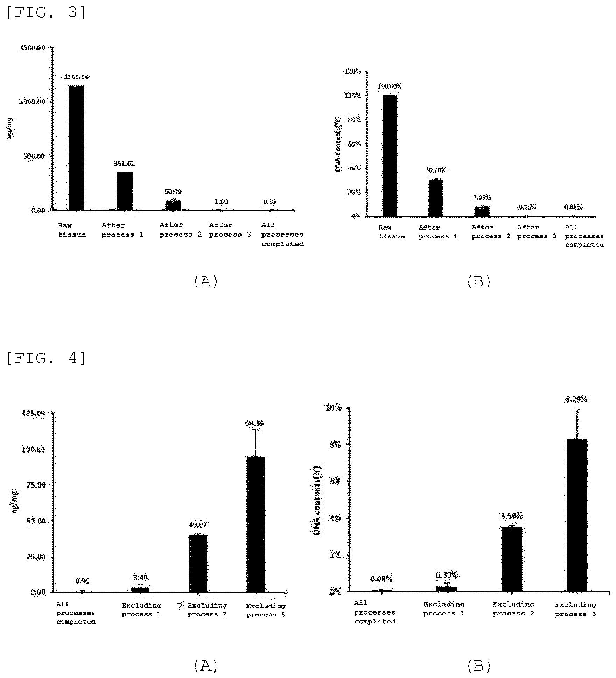

The DNA content per 1 mg of tissue after each step is shown in FIG. 3(A), and the DNA content of the tissue after each step relative to that of the original tissue is shown as a percentage in FIG. 3(B). Here, in FIGS. 3(A) and 3(B), Process 1 refers to the first decellularization step, Process 2 refers to the second decellularization step, and Process 3 refers to the secondary decellularization step.

Referring to the results of FIGS. 3(A) and 3(B), it can be confirmed that most of the DNA was removed after all of the first decellularization step, the second decellularization step, and the secondary decellularization step were performed.

**[Experimental Example 4]**

The experiment was conducted using the same method as in Experimental Example 2, except that the DNA content of protein extracts (VdECM) prepared by omitting one step each was measured using the same method as in Experimental Example 3.

The DNA content per 1 mg of protein extract (VdECM) obtained by omitting each step is shown in FIG. 4(A), and the DNA content of the tissue obtained by omitting each step relative to that of the original tissue is shown as a percentage in FIG. 4(B). Here, in FIGS. 4(A) and 4(B), Process 1 refers to the first decellularization step, Process 2 refers to the second decellularization step, and Process 3 refers to the secondary decellularization step.

Referring to FIGS. 4(A) and 4(B), it can be confirmed that DNA was substantially removed only after all of the first decellularization step, the second decellularization step, and the secondary decellularization step were performed. In particular, it was observed that omission of either the second decellularization step or the secondary decellularization step resulted in a significant decrease in DNA removal efficiency, and thus, this experiment confirmed that performing each step sequentially was more effective for DNA removal.

**[Experimental Example 5]**

A multi-step decellularized protein extract (VdECM) was prepared using the same method as in Preparation Example 1, except that the tissue was treated in the primary decellularization step with treatment solutions containing sodium hydroxide at concentrations of 0.005 M, 0.1 M, 0.2 M, and 0.3 M.

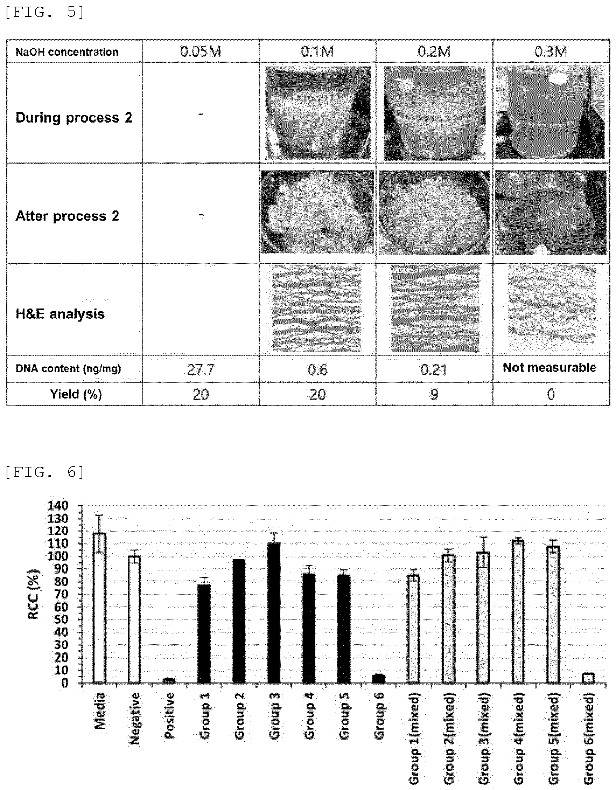

During and after the second decellularization step (second process) treatment during the experiment, tissue status was photographed and shown in FIG. 5. In addition, the DNA content per 1 mg of the final obtained protein extract (VdECM) was measured using the same method as in Experimental Example 3. The yield of the protein extract (VdECM) was calculated by expressing the content of the final obtained protein extract (VdECM) obtained as a percentage of the content of the initially introduced tissue, and the photographs of H&E-stained samples are shown in FIG. 5.

Referring to FIG. 5, it was confirmed that when the NaOH concentration was 0.05 M, the yield was good, but DNA content was high, and cell nuclei that were not removed exist within the tissue, indicating low decellularization efficiency.

In contrast, it can be confirmed that when treated with 0.1 M NaOH, the tissue structure was well maintained during the second decellularization step, the DNA content was very low at 0.6 ng/mg after the final treatment, and the yield was high at 20.8%.

When treated with 0.2 M NaOH, the DNA content was low, but some of the tissue disintegrated during the primary decellularization step, resulting in a final yield less than half of that obtained with 0.1 M NaOH treatment. When treated with 0.3 M NaOH, the tissue was confirmed to be almost completely dissolved, and it is believed that tissue harvesting is actually impossible.

From these experimental results, it could be confirmed that, in order to obtain a protein extract (VdECM) from which tissue disintegration or dissolution does not occur during the primary decellularization step, resulting in a high yield, and from which DNA within the tissue is sufficiently removed, the concentration of NaOH included in the treatment solution during the primary decellularization step, particularly the second decellularization step, should be greater than 0.05 M and less than 0.2 M.

**[Experimental Example 6]**

A multi-step decellularized protein extract (VdECM) was prepared using the same method as in the Preparation Example, except that during the first decellularization step, when the concentration of NaOH concentration included in the treatment solution was 0.005 M or 0.1 M, the tissue was treated with varying concentrations of DNase from 0.0001 to 0.003 wt%. The DNA content ratio and yield of each final obtained protein extract (VdECM) were then calculated and summarized in Table 2.

[Table 2]

| NaOH concentration | 0.05 | | | 0.1 | | |
|---|---|---|---|---|---|---|
| DNase concentration (wt%) | 0.0028 | 0.0014 | 0.0007 | 0.0002 | 0.0002 | 0.0001 |
| DNA concentration (ng/mg) | 25.7 | 38.0 | 54.7 | 0.2 | 0.3 | 0.6 |
| Yield (%) | 20.3 | 20.7 | 19.9 | 21.0 | 21.4 | 20.8 |

Referring to the results of Table 2, it was found that when the NaOH concentration was constant, as the DNase concentration increased, the DNA concentration in the final protein extract (VdECM) decreased. However, when the NaOH concentration was 0.1 M, the DNA concentration in the final obtained protein extract (VdECM) was even lower despite treatment with DNase at concentrations several to tens of times lower than at 0.05 M NaOH, and the difference in yield was considered insignificant. From these experimental results, when the NaOH concentration was 0.05 M, the DNA removal efficiency was found to be significantly lower compared to when the concentration was 0.1 M. It was thus confirmed that the NaOH concentration in the treatment solution used in the primary decellularization step should preferably exceed 0.05 M.

**[Preparation Example 2]**

The protein extract (VdECM) prepared in Preparation Example 1 was dissolved in a 0.1 N aqueous sodium hydroxide solution to have various concentrations, and then a 0.1 N aqueous acetic acid solution was added to adjust the pH to the neutral range of 7.4, thereby solubilizing the protein extract. Ultra-pure water was used as the solvent for the aqueous solution.

Drug delivery compositions containing various concentrations of the protein extract (VdECM) were prepared, as shown in Table 3 below. Here, the concentration of the amphiphilic copolymer mixtures included in drug delivery composition groups 1 to 6 was maintained at a constant 40 wt%. An amphiphilic copolymer obtained by mixing a first poloxamer and a second poloxamer at a weight ratio of 1:1 was used, where the first poloxamer was a PEO-PPO-PEO copolymer having a weight-average molecular weight of 12,600 g/mol, and the second poloxamer was a PEO-PPO-PEO copolymer having a weight-average molecular weight of 15,000 g/mol.

[Table 3]

| | Protein extract (VdECM, wt%) | Amphiphilic copolymer mixture 40 wt% |
|---|---|---|
| Group 1 | 0 | Poloxamer 1 (20 wt%) + Poloxamer 2 (20 wt%) |
| Group 2 | 1 | |
| Group 3 | 2 | |
| Group 4 | 4 | |
| Group 5 | 6 | |
| Group 6 | 12 | |

**[Experimental Example 7]**

First, a cytotoxicity test of the drug delivery composition according to changes in the concentration of the protein extract was conducted. The cytotoxicity measurement was conducted in accordance with the Common Criteria and Test Methods for Biological Safety of Medical Devices (ISO 10993-5:2009), and the elution of the sample was performed in according with ISO 10993-12. For Groups 1 to 6 of Table 3, according to the above standard test method, extracts were prepared by eluting the samples in cell culture medium at 37 °C for 24 hours under 100 rpm, and then L929 cells were seeded at $2 \times 10^5$ cells/well in 6-well plates and cultured for 24 hours, after which 2 ml of each extract was applied to the cells. Morphological changes of cells (qualitative evaluation) and cell number were measured (quantitative evaluation) over two days, and based on these results. cell viability (RCC) was confirmed using the following Equation 2.

$$RCC(\%) = \frac{cell\ number\ of\ test\ group}{cell\ number\ of\ negative\ control\ group} \times 100 \qquad \text{(Equation 2)}$$

As confirmed in FIG. 6, the cytotoxicity test results of the drug delivery composition according to the present disclosure confirmed that there was no cytotoxicity even when the concentration of the protein extract (VdECM) increased up to 6 wt%, and the RCC value also tended to increase as the concentration of the protein extract increased to 4 wt%.
However, cytotoxicity was observed when the concentration of the protein extract was as high as 12 wt%. It is presumed that cytotoxicity occurred due to due to an increase in hydrophobicity, which is a characteristic of the protein extract, and an increase in viscosity when the concentration of the protein extract was excessively high.
In FIG. 6, the "Media" refers to a blank test solution, which was eluted under the same conditions and by the same method as the test specimen but without including any test specimen. The "Negative" refers to a sample treated with a substance known not to induce a cytotoxic reaction and was used to determine the baseline cellular response. In addition, the "Positive" refers to a sample treated with a substance known to reproducibly induce cytotoxic reactions and was used to verify that the cytotoxicity test procedure was properly conducted.
It could be confirmed that, except for Group 6 containing the protein extract (VdECM) at a concentration of 12 wt%, all test groups showed RCC values of 70% or more, thereby satisfying the criterion for cytotoxicity evaluation (RCC ≥ 70%).
In the FIG. 6, the same cytotoxicity test was conducted on samples diluted by mixing each sample from Groups 1 to 6 with an equal volume of distilled water (see Groups 1 (mixed) to 6 (mixed) in FIG. 6), and the results also showed that the same results as the samples before dilution with distilled water.

**[Experimental Example 8]**

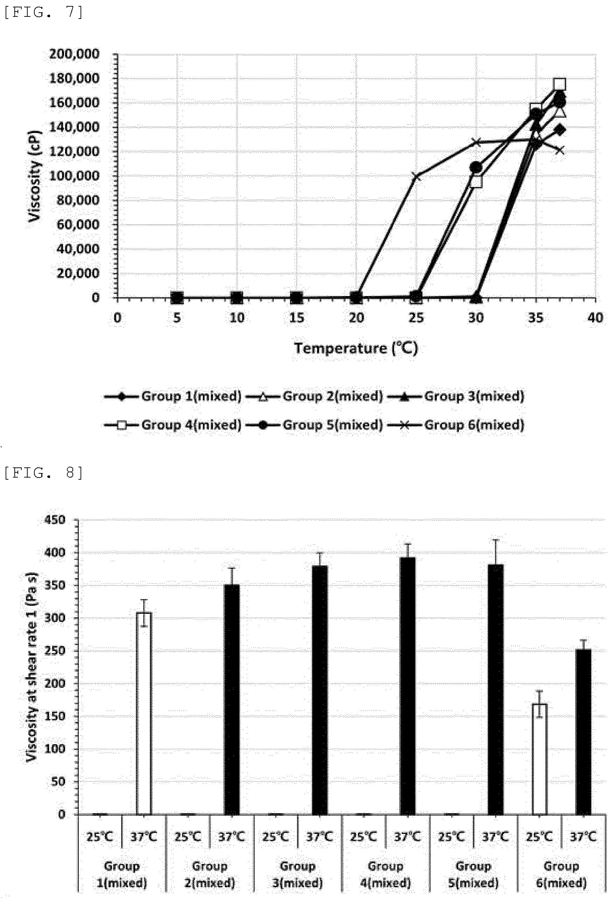

Drug delivery compositions with various protein extract (VdECM) concentrations from Table 3 (Groups 1 to 6) were mixed with equal amounts of ultrapure water (Groups 1 to 6 (mixed)), and the change in viscosity according to temperature change was measured. The results are shown in FIG. 7. That is, each sample from Groups 1 to 6 was diluted with ultrapure water at a ratio of 1:1 before viscosity measurement was made. Thus, the concentration of the protein extract in the viscosity measurement samples was half that of Groups 1 to 6.
It could be confirmed that, as the concentration of the protein extract increased, the temperature at which sol-gel transition occurred decreased, and that the maximum viscosity value increased in proportion to the concentration of the protein extract up to 6 wt%.
However, it could be confirmed that for Group 6, where the concentration of the protein extract was 12 wt%, a decrease in viscosity (a decrease in maximum viscosity value) was observed at body temperature (37°C). In particular, for Group 6, gelation began to occur at room temperature (25°C), indicating that it is undesirable for use as a drug delivery composition. This can be clearly confirmed by comparing the viscosity values of the drug delivery compositions at room temperature (25°C) and body temperature (37 °C) at a shear rate of 1/s, as shown in FIG. 8.
As confirmed in the results of FIG. 8, it was found that the concentration of the original solution before dilution of the protein extract was less than 12 wt%, and more preferably 6 wt% or less, the sol-gel transition proceeded effectively as the temperature changed from room temperature to body temperature. Conversely, it was also found that when the concentration of the protein extract was excessively increased, the sol-gel transition between room temperature and body temperature did not proceed smoothly.

**[Experimental Example 9]**

To determine whether the drug delivery composition according to the present disclosure could stably remain at the site of injection *in vivo* after injection into the body, the adhesive strength was measured. For Groups 1 to 5, excluding Group 6 which was previously identified as undesirable in Experimental Example 8, a probe was vertically pressed onto each sample with a constant force. Subsequently, the changes in the measured normal stress values were observed during the process of vertically lifting and debonding the probe from each sample from Groups 1 to 5.

The samples used for adhesive strength measurement were also prepared by diluting each sample from Groups 1 to 5 with an equal amount of ultrapure water at a ratio of 1:1, similar to the viscosity measurement process described above. Therefore, the concentration of the protein extract in the samples used for the actual adhesive strength measurement experiment was half that listed in Table 3.

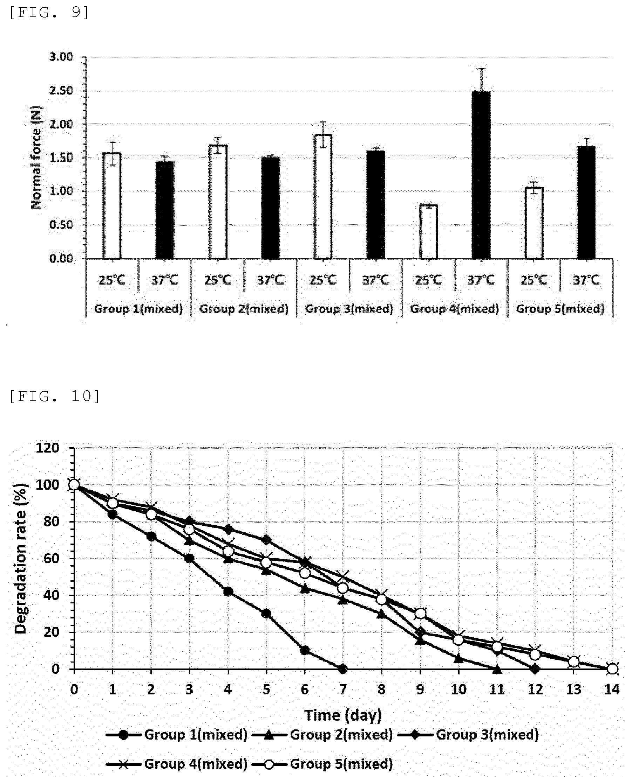

This adhesive strength measurement experiment was conducted at two temperatures, room temperature (25°C) and body temperature (37°C), similar to the viscosity measurement in Experimental Example 8 described above, and the results are shown in FIG. 9.

As confirmed in the results of FIG. 9, it was found that when the concentration of the protein extract (VdECM) was in the range of 2 wt% or less (Groups 1 to 3), the adhesive strength at body temperature (37°C) decreased compared to room temperature (25°C), but when the concentration of the protein extract was in the range of 4 to 6 wt% (Groups 4 and 5), adhesive strength increased at body temperature compared to room temperature. It was also found that Group 4, in which the concentration of the protein extract (VdECM) was 4 wt% (the concentration of protein extract included in the actual sample was about 2 wt%), exhibited the highest adhesive strength at body temperature.

**[Experimental Example 10]**

Next, when the drug delivery composition according to the present disclosure underwent a phase change to form a gel, the phase stability of the resulting gel formulation was confirmed. 5 g of drug delivery compositions containing various concentrations of the protein extract from Groups 1 to 5, as shown in Table 3, were mixed with 5 g of ultrapure water, placed in tubes, and then gelled at body temperature (37°C) for about 30 minutes.

Thereafter, 2.5 g of phosphate buffered saline (PBS) was additionally added to the tube and stored at 37°C while being shaken at about 50 rpm. After 24 hours, the tubes were inverted to measure the remaining height of the drug delivery system, replaced with fresh PBS, and stored under the same conditions (37°C, 50 rpm).

This process was repeated every 24 hours, during which the height of the gel-form drug delivery system was repeatedly measured every 24 hours until the remaining drug delivery system, the gel, was completely degraded. The results are summarized in FIG. 10.

As confirmed in the results of FIG. 10, the gel of the drug delivery composition, which did not contain the protein extract (VdECM) (Group 1) was completely degraded within 7 days, whereas Group 2 was degraded in 11 days, Group 3 was degraded in 12 days, and Groups 4 and 5 were completely degraded in 14 days.

This indicates that by including the protein extract in the drug delivery composition at a predetermined concentration range, gel stability after phase change may be enhanced, and that when inserted into the body, the drug delivery system can maintain its gel form for a long period time, thereby exhibiting excellent sustained release properties by slowly releasing the drug contained in the drug delivery system for a long period time.

**[Experimental Example 11]**

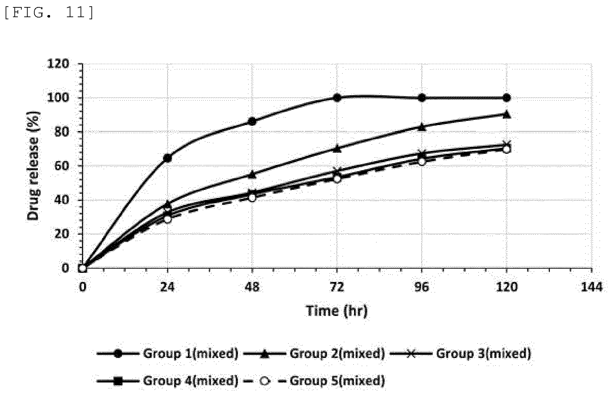

To verify the actual drug release behavior of the drug delivery compositions according to the present disclosure, drug delivery compositions from Groups 1 to 5 were mixed with the drug, Ropivacaine HCl, at a ratio of 1:1, and the amount of drug released was measured every 24 hours for 120 hours (5 days). The results are summarized in FIG. 11.

As confirmed in the results of FIG. 11, in Group 1, which did not contain the protein extract, the drug, Ropivacaine HCl, was completely released within 72 hours (100% drug release) .

In contrast, it was confirmed that Groups 2 to 5, which contained the protein extract, released the drug more slowly for a long period of time compared to Group 1, which did not contain the protein extract. Among them, Groups 4 and 5 were found to exhibit the most excellent sustained-release properties.

Considering that the known *in vivo* half-life of a single drug, Ropivacaine HCl, is 1.8 + 0.7 hours for intravenous injection and 4.2 $\pm$ 1 hours for epidural injection, it was found that, when the drug was applied using the drug delivery composition according to the present disclosure, the drug could be stably supplied into the body for a long period of time, compared to when injected using a conventional injection.

[Industrial Applicability]

The drug delivery composition according to the present disclosure comprises an amphiphilic block copolymer including a mixture of a first poloxamer and a second poloxamer having different molecular weights; and a protein extract (VdECM) containing elastin and collagen. The composition is capable of being easily administered into the body in a liquid state, forming a stable drug delivery structure through a phase transition induced by body temperature, thereby effectively releasing a drug component in the body for a long period of time, being biodegradable and absorbable in the body, and promoting tissue regeneration. Therefore, the composition exhibits industrial applicability.

**Claims**

1. A drug delivery composition for promoting tissue regeneration, comprising:

   an amphiphilic block copolymer including a mixture of a first poloxamer and a second poloxamer having different molecular weights; and
   a protein extract (VdECM) containing elastin and collagen.

2. The drug delivery composition for promoting tissue regeneration of claim 1, wherein the amphiphilic block copolymer is in a sol phase at room temperature and undergoes a phase transition to a gel phase at body temperature.

3. The drug delivery composition for promoting tissue regeneration of claim 1, wherein the drug delivery composition for promoting tissue comprises 0.05 to 10 wt% of the protein extract (VdECM), 1 to 40 wt% of the first poloxamer, 1 to 40 wt% of the second poloxamer, and the balance of ultrapure water.

4. The drug delivery composition for promoting tissue regeneration of claim 1, wherein the first poloxamer has a structure of polyethylene oxide (PEO)-polypropylene oxide (PPO)-polyethylene oxide (PEO) and has a weight-average molecular weight of 5,000 to 15,000.

5. The drug delivery composition for promoting tissue regeneration of claim 1, wherein the second poloxamer has a structure of polyethylene oxide (PEO)-polypropylene oxide (PPO)-polyethylene oxide (PEO) and has a weight-average molecular weight of 8,000 to 20,000.

6. The drug delivery composition for promoting tissue regeneration of claim 1, wherein the protein extract (VdECM) is obtained by the following steps:

   a pretreatment step of preparing and pretreating tissue of non-human mammal;
   a first inactivation step of inactivating viruses contained in the pretreated tissue using an alcohol;
   a decellularization step of removing cells from virus-inactivated tissue; and
   a second inactivation step of inactivating viruses contained in the decellularized tissue using an acid,
   wherein a DNA content of the tissue that had undergone the second inactivation step is 50 ng/mg or less, and
   a reduction rate (L) of the elastin content in the tissue that had undergone the pretreatment step to the second inactivation step is 20% or less.

7. The drug delivery composition for promoting tissue regeneration of claim 6, wherein the decellularization step includes:

   a primary decellularization step of removing cells from the virus-inactivated tissue using an alkaline aqueous solution; and
   a secondary decellularization step of removing cells by enzymatic treatment of the primarily decellularized tissue.

8. The drug delivery composition for promoting tissue regeneration of claim 7, wherein the primary decellularization step includes a first decellularization step performed using a mixture of n-PrOH and NaOH; and a second decellularization step performed using an aqueous sodium hydroxide solution having a concentration of greater than 0.05 M and less than 0.2 M.

9. The drug delivery composition for promoting tissue regeneration of claim 6, wherein the tissue of a non-human mammal is one or more selected from the group consisting of blood vessels, ligaments, and tendons derived from a mammal.

10. The drug delivery composition for promoting tissue regeneration of claim 7, wherein the secondary decellularization step is performed using DNase.

11. A kit for drug delivery filled with the drug delivery composition for promoting tissue regeneration of any one of claims 1 to 10.

12. A drug delivery system for promoting tissue regeneration, comprising a mixture of the drug delivery composition for promoting tissue regeneration of any one of claims 1 to 10 and a drug at a weight ratio of 1:1.

[FIG. 1]

(A)                                                    (B)

[FIG. 2]

[FIG. 3]

(A)

(B)

[FIG. 4]

(A)

(B)

[FIG. 5]

| NaOH concentration | 0.05M | 0.1M | 0.2M | 0.3M |
|---|---|---|---|---|
| During process 2 | - | | | |
| Atter process 2 | - | | | |
| H&E analysis | | | | |
| DNA content (ng/mg) | 27.7 | 0.6 | 0.21 | Not measurable |
| Yield (%) | 20 | 20 | 9 | 0 |

[FIG. 6]

[FIG. 7]

[FIG. 8]

[FIG. 9]

[FIG. 10]

[FIG. 11]

## INTERNATIONAL SEARCH REPORT

| International application No. |
|---|
| **PCT/KR2024/000965** |

| A. | CLASSIFICATION OF SUBJECT MATTER |
|---|---|

**A61K 9/00**(2006.01)i; **A61K 9/06**(2006.01)i; **A61K 47/10**(2006.01)i; **A61K 38/39**(2006.01)i; **A61P 43/00**(2006.01)i

According to International Patent Classification (IPC) or to both national classification and IPC

| B. | FIELDS SEARCHED |
|---|---|

Minimum documentation searched (classification system followed by classification symbols)

A61K 9/00(2006.01); A61K 31/496(2006.01); A61K 47/10(2006.01); A61K 47/32(2006.01); A61K 47/34(2006.01); A61K 47/36(2006.01); A61K 9/16(2006.01); A61K 9/70(2006.01); C08G 65/26(2006.01); C08G 65/48(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 양친매성(amphiphilic), 공중합체(copolymer), 폴록사머 (poloxamer), 혈관유래세포외기질(vessel-derived decellularized extracellular matrices, VdECM), 조직 재생(tissue regeneration), 약물 전달(drug delivery), 상변화(phase transition), 엘라스틴(elastin), 콜라겐(collagen)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | KR 10-2144615 B1 (JANG, Geon-Ju) 13 August 2020 (2020-08-13)<br>See abstract; claims 1-2 and 6-7; and paragraphs [0008]-[0009]. | 1-12 |
| A | KR 10-2009-0057674 A (KOREA ADVANCED INSTITUTE OF SCIENCE AND TECHNOLOGY) 08 June 2009 (2009-06-08)<br>See abstract; and claims 1-2 and 10-12. | 1-12 |
| A | KR 10-2022-0019366 A (KOREA INSTITUTE OF SCIENCE AND TECHNOLOGY) 17 February 2022 (2022-02-17)<br>See entire document. | 1-12 |
| A | KR 10-1695440 B1 (KBNP, INC.) 11 January 2017 (2017-01-11)<br>See entire document. | 1-12 |

| ✓ Further documents are listed in the continuation of Box C. | ✓ See patent family annex. |
|---|---|

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **30 April 2024** | **01 May 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2024/000965**

## C. DOCUMENTS CONSIDERED TO BE RELEVANT

| Category* | Citation of document, with indication, where appropriate, of the relevant passages | Relevant to claim No. |
|---|---|---|
| A | EP 2448567 B1 (BENDER ANALYTICAL HOLDING B.V.) 13 March 2013 (2013-03-13)<br>See entire document. | 1-12 |
| A | TOMASINA, C. et al. Bioprinting Vasculature: Materials, Cells and Emergent Techniques. Materials. 2019, vol. 12, document no. 2701, pp. 1-42.<br>See entire document. | 1-12 |

**EP 4 666 999 A1**

**INTERNATIONAL SEARCH REPORT**
**Information on patent family members**

International application No.

**PCT/KR2024/000965**

| Patent document cited in search report | | | Publication date (day/month/year) | Patent family member(s) | | | Publication date (day/month/year) |
|---|---|---|---|---|---|---|---|
| KR | 10-2144615 | B1 | 13 August 2020 | KR | 10-2020-0017765 | A | 19 February 2020 |
| KR | 10-2009-0057674 | A | 08 June 2009 | KR | 10-0953170 | B1 | 20 April 2010 |
| KR | 10-2022-0019366 | A | 17 February 2022 | KR | 10-2431839 | B1 | 12 August 2022 |
| KR | 10-1695440 | B1 | 11 January 2017 | KR | 10-2016-0033080 | A | 22 March 2016 |
| EP | 2448567 | B1 | 13 March 2013 | CN | 102481264 | A | 30 May 2012 |
| | | | | CN | 102481264 | B | 22 April 2015 |
| | | | | EP | 2448567 | A1 | 09 May 2012 |
| | | | | JP | 2012-532099 | A | 13 December 2012 |
| | | | | JP | 5666574 | B2 | 12 February 2015 |
| | | | | KR | 10-1701544 | B1 | 01 February 2017 |
| | | | | KR | 10-2012-0107064 | A | 28 September 2012 |
| | | | | US | 2012-0183606 | A1 | 19 July 2012 |
| | | | | US | 8642080 | B2 | 04 February 2014 |
| | | | | WO | 2011-002285 | A1 | 06 January 2011 |

Form PCT/ISA/210 (patent family annex) (July 2022)